(19) Europäisches Patentamt
European Patent Office
Office européen des brevets

(11) Veröffentlichungsnummer : **0 373 384 B1**

# (12) EUROPÄISCHE PATENTSCHRIFT

(45) Veröffentlichungstag der Patentschrift : **28.10.92 Patentblatt 92/44**

(51) Int. Cl.$^5$ : **A61K 6/083,** C08F 20/28, C08F 14/18

(21) Anmeldenummer : **89121422.3**

(22) Anmeldetag : **20.11.89**

## (54) Perfluoralkylgruppen aufweisende (Meth-)acrylsäureester, deren Herstellung und ihre Verwendung in der Dentaltechnik.

(30) Priorität : **10.12.88 DE 3841617**

(43) Veröffentlichungstag der Anmeldung : **20.06.90 Patentblatt 90/25**

(45) Bekanntmachung des Hinweises auf die Patenterteilung : **28.10.92 Patentblatt 92/44**

(84) Benannte Vertragsstaaten : **AT BE CH DE ES FR GB GR IT LI LU NL SE**

(56) Entgegenhaltungen :
**US-A- 4 508 916**
**US-A- 4 612 356**
**US-A- 4 616 073**
**US-A- 4 644 043**

(73) Patentinhaber : **Th. Goldschmidt AG**
**Goldschmidtstrasse 100 Postfach 101461**
**W-4300 Essen 1 (DE)**
Patentinhaber : **GDF GESELLSCHAFT FÜR DENTALE FORSCHUNG UND INNOVATIONEN GMBH**
**Dieselstrasse 6**
**W-6365 Rosbach (DE)**

(72) Erfinder : **Fock, Jürgen, Dr.**
**Mörsenbroicher Weg 114**
**W-4000 Düsseldorf 30 (DE)**
Erfinder : **Hahn, Günter, Dr.**
**Eigene Scholle 35**
**W-4330 Mülheim/Ruhr (DE)**
Erfinder : **Wagenknecht, Günther**
**Bahnhofstrasse 7a**
**W-6363 Echzell (DE)**

EP 0 373 384 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Fluoralkylgruppen aufweisende (Meth-)acrylsäureester, deren Herstellung und Verwendung in der Dentaltechnik. Sie betrifft insbesondere solche Verbindungen, die als härtbare Polymerisate im Dentalbereich zur Unterfütterung von Zahnprothesen geeignet sind.

Der Ausdruck (Meth-)acrylsäureester soll dabei bedeuten, daß sowohl Methacrylsäureester wie Acrylsäureester gleichermaßen von der Erfindung erfaßt werden.

Aus der Literatur sind fluorhaltige monomere und oligomere (Meth-)acrylate bekannt. Sie werden zur Herstellung dentaler Prothesen- und Füllungsmaterialien verwendet und verleihen diesen verminderte Wasseraufnahme und verringerte Löslichkeit.

Beispielsweise ist im J.Dent.Res., 58, 1981 bis 1986, die Verwendung von 1,1,5-Trihydro-octafluoropentyl-methacrylat als polymerisierbarer Bestandteil in Zahnfüllmassen beschrieben. Sodann sind fluorhaltige Phenylcarbinol-acrylate, wie 1,1,1,3,3,3-Hexafluor-2-phenyl-2-acryloyloxy-propan aus Org.Coat.Plast.Chem. 42, 204 bis 207, 1980, bekannt.

Weiter werden in der US-PS 4 356 296 ähnliche Verbindungen und ihre Verwendung auf dem Dentalgebiet beschrieben. Die US-PS 4 616 072 offenbart Perfluoralkylmonomethacrylate als hydrophobe Copolymerisate für dentale Füllungsmaterialien. Ebenfalls für die restaurative Zahmedizin dienen die in den EP-A2-0 201 031 und 0 201 778 offenbarten Monomeren mit substituiertem Bis-phenyltetrafluorethan.

Diese vorbekannten Monomeren weisen den Nachteil auf, daß bei ihrer Aushärtung im wesentlichen hartspröde Polymerisate entstehen, was ihre Verwendungsmöglichkeit in der Dentaltechnik stark einschränkt.

Der Erfindung liegt die Aufgabe zugrunde, härtbare Monomere für den Einsatz in der Dentaltechnik bereitzustellen, welche neben einer reduzierten Löslichkeit und einer erhöhten mechanischen Festigkeit des ausgehärteten Endproduktes insbesondere als Unterfüllungsmaterial für Dentalprothesen mit einer erhöhten Haftung zu bereits ausgehärtetem Polymethylmethacrylat verwendet werden können.

Gegenstand der Erfindung sind zunächst neue makromonomere Fluoralkylgruppen aufweisende (Meth-)acrylsäureester der allgemeinen Formel

$$R^1-(C_nF_{2n}-)(CH_2-)_aO-(C_mH_{2m}O-)_b(\underset{\underset{O}{\|}}{C}-NH-(CH_2-)_cO-)\underset{\underset{O}{\|}}{C}-\underset{\underset{R^2}{|}}{C}=CH_2 \qquad \mathrm{I}$$

wobei $R^1$ gleich oder verschieden und ein Wasserstoff- oder Fluorrest ist,

$R^2$ gleich oder verschieden und ein Wasserstoff- oder Methylrest ist,
a einen Wert von 0, 1, 2, 3 oder 4,
c einen Wert von 2, 3 oder 4,
b einen durchschnittlichen Wert von 2 bis 30,
n einen durchschnittlichen Wert von 4 bis 12 und
m einen durchschnittlichen Wert von 3 bis 14 hat.

Die vorstehende Formel I ist als durchschnittliche, allgemeine Formel eines Makromonomerengemisches zu verstehen. Dabei unterscheiden sich die einzelnen Individuen insbesondere durch die Anzahl ihrer Oxyalkylengruppen, die mit dem durchschnittlichen Wert von b als Maximum einer Schulz-Flory-Verteilung entspricht oder dieser angenähert ist.

Die Kettenlänge der Fluoralkylgruppe wird durch den Index n bestimmt. n hat dabei einen durchschnittlichen Wert von 4 bis 12. Ist der zugrunde liegende Alkohol $R^1C_nF_{2n}-(CH_2-)_aOH$ eine einzelne Verbindung, ist der Wert von n absolut und entspricht einer ganzen Zahl von 4 bis 12. Bevorzugt sind Verbindungen mit einem durchschnittlichen oder absoluten Wert von n = 6 bis 10.

Die Hydroxylfunktion des Fluoralkohols kann durch eine oder mehrere $CH_2$-Gruppe(n) von der Perfluoralkylgruppe getrennt sein. Die Anzahl solcher $CH_2$-Gruppen ergibt sich aus dem Wert von a. a kann eine ganze Zahl, nämlich 0, 1, 2, 3 oder 4 sein.

Der Index m der Oxyalkylengruppe $C_mH_{2m}O-$ hat einen durchschnittlichen Wert von 3 bis 14. Die Oxyalkylengruppe kann dabei die Struktur

$$CH_2\underset{\underset{R^3}{|}}{C}HO- ,$$

wobei $R^3$ ein Alkylrest mit 1 bis 12 Kohlenstoffatomen ist, oder $CH_2CH_2CH_2CH_2O-$ haben, wenn für die Herstellung des fluorierten Alkanolpolyethers Tetrahydrofuran verwendet wird.

Beispiele von geeigneten Oxyalkylengruppen sind der Oxypropylen-, Oxybutylen-, Oxyoctylen-, Oxydecylen- und Oxydodecylenrest. Die erfindungsgemäßen Verbindungen können innerhalb des individuellen Moleküls gleiche oder verschiedene Oxyalkylengruppen aufweisen, so daß der Index m in dem einen Fall als absoluter, in dem anderen Fall als durchschnittlicher Zahlenwert zu verstehen ist. Liegen im gleichen Molekül verschiedene Oxyalkylengruppen nebeneinander - statistisch oder blockweise angeordnet - vor, soll das Molekül frei von Oxyethylengruppen sein. Weist das Molekül nur gleiche Oxyalkylengruppen auf, ist also der Wert m als absolut zu verstehen, folgt aus der Untergrenze von m = 3, daß Oxyethylengruppen ausgeschlossen sind.

Verbindungen, die Perfluoralkylgruppen enthalten, sind in der Regel in üblichen Lösungsmitteln schwer löslich oder unlöslich und deshalb schwierig zu handhaben.

Durch die Anwesenheit von Oxyalkylengruppen mit längerkettigen Resten $R^3$ können die Eigenschaften der erfindungsgemäßen Verbindungen, insbesondere nach der Polymerisation, wie Hydrophobie und elastisch/plastisches Verhalten, sowie ihre Löslichkeit beeinflußt und dem Anwendungszweck angepaßt werden.

Die Anzahl der Oxyalkylengruppen ergibt sich aus dem Wert von b und beträgt im Mittel 2 bis 30. Bevorzugt ist ein b-Wert von 5 bis 20.

Besonders bevorzugte erfindungsgemäße Verbindungen sind solche, bei denen im durchschnittlichen Molekül mindestens 50 Mol-% der Oxyalkylen-Einheiten Oxypropylen- und/oder Oxybutylen-Einheiten sind und der durchschnittliche Wert von m bei den übrigen Oxyalkylen-Einheiten 5 bis 14 beträgt. Es hat sich gezeigt, daß diese Verbindungen für den vorgesehenen Verwendungszweck besonders geeignet sind.

Insbesondere bevorzugt sind Verbindungen mit dem Kennzeichen, daß im durchschnittlichen Molekül mindestens 90 Mol-% der Oxyalkylen-Einheiten Oxypropylen- und/oder Oxybutylen-Einheiten sind und der durchschnittliche Wert von m bei den übrigen Oxyalkylen-Einheiten 5 bis 14 beträgt.

Verbindungen, bei denen die Oxyalkylen-Einheiten ausschließlich aus Oxypropylen- und/oder Oxybutylen-Einheiten bestehen, verbinden hervorragende Eigenschaften mit niedrigem Kosteneinsatz.

Die Synthese von alkoxylierten Perfluoralkanolen erfolgt zweckmäßig durch kationische Polyaddition unter Verwendung von Lewis-Säuren, wie z.B. Bortrifluorid, Bortrifluorid-Etherat und Zinntetrachlorid.

Ein weiterer Gegenstand vorliegender Erfindung besteht in dem Verfahren zur Herstellung dieser Verbindungen. Dieses Verfahren ist dadurch gekennzeichnet, daß man einen Polyoxyalkylenmonoether der allgemeinen Formel

$$R^1\text{-}(C_nF_{2n}\text{-})(CH_2\text{-})_aO\text{-}(C_mH_{2m}O\text{-})_bH$$

mit einem Isocyanat der allgemeinen Formel

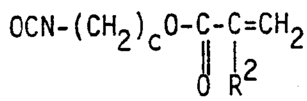

in an sich bekannter Weise bei Temperaturen eines Bereiches von 20 bis 100°C, gegebenenfalls in Gegenwart eines gegenüber Isocyanatgruppen inerten Lösungsmittels und gegebenenfalls in Gegenwart eines an sich bekannten Katalysators für die Reaktion der Isocyanatgruppe mit der Hydroxylgruppe umsetzt.

Die Indices haben die bereits angegebene Bedeutung.

Im allgemeinen läuft die Reaktion bereits bei niedrigen Temperaturen, wie z.B. Raumtemperatur, ab. Mäßiges Erwärmen bis auf etwa 100°C beschleunigt den Ablauf der Reaktion. Auf ein Lösungsmittel kann in den meisten Fällen verzichtet werden. Will man aus verfahrenstechnischen Gründen jedoch nicht auf ein Lösungsmittel verzichten, eignen sich solche Lösungsmittel, die gegenüber Isocyanatgruppen inert sind, wie z.B. Toluol oder Xylol. Empfehlenswert ist die Verwendung eines Katalysators für die Reaktion des Isocyanates mit dem Alkohol. Derartige Katalysatoren sind dem Fachmann geläufig und der Literatur zu entnehmen. Besonders bevorzugt sind Zinnkatalysatoren, wie Dibutylzinndilaurat und Zinnoctoat.

Zur Vermeidung einer vorzeitigen Polymerisation ist den Reaktionsansätzen eine hinreichende Menge eines geeigneten Polymerisationsinhibitors zuzusetzen. Geeignete Polymerisationsinhibitoren sind Hydrochinon, Hydrochinonmonomethylether oder t-Butylcatechol.

Die erhaltenen Verbindungen zeichnen sich durch einerseits ihren makromolekularen Charakter und andererseits ihre ungesättigte Endgruppe aus, die wiederum zur Polymerisation befähigt ist; in der Fachwelt nennt man deshalb diese Verbindungen auch Makromonomere.

Ein weiterer Gegenstand der Erfindung besteht in der Verwendung der erfindungsgemäßen Verbindungen als härtbare Monomere im Dentalbereich. Die erfindungsgemäßen Verbindungen werden hierfür mit in der

Dentaltechnik üblichen Zusatzstoffen compoundiert. Solche Zusatzstoffe können Füllmittel, wie - insbesondere hydrophobierte - Glaskeramik, feinteilige Kieselsäure oder Pigmente oder Modifizierungsmittel sein. Die Modifizierungsmittel dienen dazu, bestimmte anwendungstechnisch wichtige Eigenschaften, wie Elastizität, Reißfestigkeit, Alterungsbeständigkeit, Verträglichkeit, zu optimieren.

Weitere geeignete Modifizierungsmittel sind Divinylbenzol, Ethylenglykoldimethacrylat, Butandioldimethacrylat, Trimethylolpropantrimethacrylat und Pentaerythrittetramethacrylat.

Den Zubereitungen werden ferner Katalysatoren für die strahlungsinduzierte Polymerisation, wie Benzildimethylketal, 2,3-Bornandion, Dimethylaminobenzolsulfansäure-bis-allylamid, Benzophenon, Diethoxyacetophenon, in Mengen von 0,1 bis 3 Gew.-% zugesetzt. Die Härtung der Zubereitung erfolgt mit Hilfe in der Dentaltechnik üblicher Lampen, deren Strahlung eine Wellenlänge von 200 bis 550 nm hat.

Die Härtung kann auch mit peroxidischen Katalysatoren oder Initiatoren bei erhöhten Temperaturen durchgeführt werden. Hierzu werden Peroxide, wie Dibenzoylperioxid, eingesetzt. Bei niedrigen Temperaturen ist eine Vernetzung mit Hilfe von Redox-Initiatoren möglich. Beispiel eines solchen Redox-Initiators ist das System Dibenzoylperoxid/N,N-Dihydroxyethyl-p-toluidin.

Die Compoundierung mit diversen Additiven und die Aushärtung der erfindungsgemäßen (Meth-) acrylsäureester zu anwendungstechnisch optimalen Dentalprodukten geschieht in an sich bekannter Weise und kann bei spielsweise den oben zitierten Publikationen, insbesondere den EP-A2-0 201 031 und 0 201 778, entnommen werden.

## Beispiel

a) Herstellung eines α-Hydroxy-ω-perfluoralkylalkanolpolyethers (nicht erfindungsgemäß)

170 g (ca. 0,37 Mol) Perfluoroctylethanol und 9,2 g Zinntetrachlorid werden in einem Druckreaktor unter Reinstickstoff auf 60°C aufgeheizt und dazu über 3 h 163 g (ca. 2,8 Mol) Propylenoxid gegeben. Nach einer Nachreaktion mit der Dauer von 0,5 h wird abgekühlt. Die an einer Probe des Produktes ermittelte Epoxidzahl von 0,01 zeigt an, daß die Reaktion weitgehend beendet ist. Das Produkt wird mit 25 vol.-%igem Ammoniak neutralisiert, das Wasser bei 80°C und 10 Torr abdestilliert und schließlich in Gegenwart eines Filterhilfsmittels filtriert.

Die naßanalytisch ermittelte Hydroxylzahl beträgt 63, was bei einer angenommenen Funktionalität von 1 einem Molekulargewicht von ca. 890 entspricht.

b) Herstellung eines α-Methacryloyl-ω-perfluoralkanolpolyetherurethans (erfindungsgemäß)

445 g (ca. 0,5 Mol) des α-Hydroxy-ω-perfluoralkanolpolyethers aus a) werden mit 0,3 g 2,6-Di-tert.-Butylkresol versetzt und auf 45 bis 50° C erwärmt. Unter stetigem Rühren werden nun 77,5 g (ca. 0,5 Mol) Isocyanatoethylmethacrylat, dem ein Tropfen Dibutylzinndilaurat zugesetzt wurde, zugetropft. Die Reaktionstemperatur sollte 60° C nicht überschreiten. Der Reaktionsverlauf wird im IR-Spektrometer bis zum restlosen Verschwinden der NCO-Absorptionsbande verfolgt, die dafür erforderliche Zeit beträgt etwa 12 h, kann aber durch Hinzufügen von Zinn(II)octoat verkürzt werden. Die Ausbeute beträgt annähernd 100 %. Das osmometrisch ermittelte Molekulargewicht beträgt 1040 und entspricht damit dem theoretisch ermittelten Molekulargewicht.

c) Herstellung eines weichbleibenden Unterfütterungsmaterials für Dentalprothesen

60 Gew.-Teile der erfindungsgemäßen Verbindung aus b) werden mit 30 Gew.-Teilen 2,2,3,3-Tetrafluoropropylmethacrylat und 10 Gew.-Teilen 2,2,3,3,4,4-Hexafluor-1,5-pentandiol-dimethacrylat gemischt. Der Mischung werden 1,5 Gew.-% Di-Benzoylperoxid zugesetzt.

Zu dieser Lösung werden durch intensives Mischen unter Vakuum im Labor-Planetenmischer 35 Gew.-Teile hydrophobe Kieselsäure zugegeben. Es entsteht eine klar-transparente Paste, die sowohl im Preßals auch im Injektions-Verfahren als weiches Unterfütterungsmaterial für Dentalprothesen verwendet werden kann. Die Aushärtung erfolgt bei 70 bis 90°C im Wasserbad innerhalb von 1 bis 3 h.

Tabelle

| Beispiel | Wasseraufnahme mg/cm² | Shore A Härte 37°C |
|---|---|---|
| c) | 0,80 ± 0,20 | 30 - 35 |
| Handelsprodukt auf Silikonbasis | 1,70 ± 0,80 | 30 - 35 |
| Handelsprodukt auf Basis weichgemachter Methacrylate | 6,90 ± 1,20 | 47 - 50 |

**Patentansprüche**

**Patentansprüche für folgende Vertragsstaaten : AT, BE, CH, DE, FR, GB, GR, IT, LI, LU, NL, SE**

1. Makromonomere Fluoralkylgruppen aufweisende (Meth-)acrylsäureester der allgemeinen Formel

$$R^1-(C_nF_{2n}-)(CH_2-)_aO-(C_mH_{2m}O-)_b(\underset{\underset{O}{\|}}{C}-NH-(CH_2-)_cO-)\underset{\underset{O}{\|}}{C}-\underset{\underset{R^2}{|}}{C}=CH_2$$

wobei $R^1$ gleich oder verschieden und ein Wasserstoff- oder Fluorrest ist,
$R^2$ gleich oder verschieden und ein Wasserstoff- oder Methylrest ist,
a einen Wert von 0, 1, 2, 3 oder 4,
c einen Wert von 2, 3 oder 4,
b einen durchschnittlichen Wert von 2 bis 30,
n einen durchschnittlichen Wert von 4 bis 12 und
m einen durchschnittlichen Wert von 3 bis 14 hat.

2. (Meth-)acrylsäureester nach Anspruch 1, dadurch gekennzeichnet, daß im durchschnittlichen Molekül mindestens 50 Mol-% der Oxyalkylen-Einheiten Oxypropylen- und/oder Oxybutylen-Einheiten sind und der durchschnittliche Wert von m bei den übrigen Oxyalkylen-Einheiten 5 bis 14 beträgt.

3. (Meth-)acrylsäureester nach Anspruch 2, dadurch gekennzeichnet, daß im durchschnittlichen Molekül mindestens 90 Mol-% der Oxyalkylen-Einheiten Oxypropylen- und/oder Oxybutylen-Einheiten sind und der durchschnittliche Wert von m bei den übrigen Oxyalkylen-Einheiten 5 bis 14 beträgt.

4. (Meth-)acrylsäureester nach Anspruch 3, dadurch gekennzeichnet, daß die Oxyalkylen-Einheiten ausschließlich aus Oxypropylen- und/oder Oxybutylen-Einheiten bestehen.

5. Verfahren zur Herstellung von (Meth-)acrylsäureestern nach Anspruch 1, dadurch gekennzeichnet, daß

man einen Polyoxyalkylenmonoether der allgemeinen Formel

$$R^1\text{-}(C_nF_{2n}\text{-})(CH_2\text{-})_aO\text{-}(C_mH_{2m}O\text{-})_bH$$

mit einem Isocyanat der allgemeinen Formel

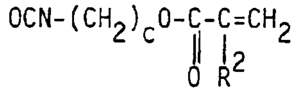

wobei die Indices die bereits angegebene Bedeutung haben, in an sich bekannter Weise bei Temperaturen eines Bereiches von 20 bis 100°C, gegebenenfalls in Gegenwart eines gegenüber Isocyanatgruppen inerten Lösungsmittels und gegebenenfalls in Gegenwart eines an sich bekannten Katalysators für die Reaktion der Isocyanatgruppe mit der Hydroxylgruppe umsetzt.

6. Verwendung von (Meth-)acrylsäureestern nach einem der Ansprüche 1 bis 4 als härtbare Monomere im Dentalbereich.

7. Verwendung von (Meth-)acrylsäureestern nach Anspruch 6 als Unterfütterungsmaterial für Zahnprothesen.

**Patentansprüche für folgenden Vertragsstaat: ES**

1. Verfahren zur Herstellung von makromonomeren Fluoralkylgruppen aufweisenden (Meth-)acrylsäureestern der allgemeinen Formel

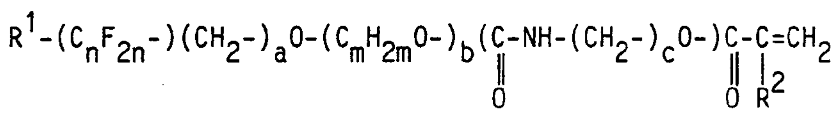

wobei $R^1$ gleich oder verschieden und ein Wasserstoff- oder Fluorrest ist,
$R^2$ gleich oder verschieden und ein Wasserstoff- oder Methylrest ist,
a einen Wert von 0, 1, 2, 3 oder 4,
c einen Wert von 2, 3 oder 4,
b einen durchschnittlichen Wert von 2 bis 30,
n einen durchschnittlichen Wert von 4 bis 12 und
m einen durchschnittlichen Wert von 3 bis 14 hat,
dadurch gekennzeichnet, daß man einen Polyoxyalkylenmonoether der allgemeinen Formel

$$R^1\text{-}(C_nF_{2n}\text{-})(CH_2\text{-})_aO\text{-}(C_mH_{2m}O\text{-})_bH$$

mit einem Isocyanat der allgemeinen Formel

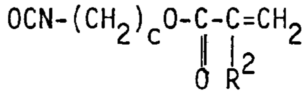

wobei die Indices die bereits angegebene Bedeutung haben, in an sich bekannter Weise bei Temperaturen eines Bereiches von 20 bis 100 °C, gegebenenfalls in Gegenwart eines gegenüber Isocyanatgruppen inerten Lösungsmittels und gegebenenfalls in Gegenwart eines an sich bekannten Katalysators für die Reaktion der Isocyanatgruppe mit der Hydroxylgruppe umsetzt.

2. Verfahren nach Anspruch 1, dadurch gekennzeichnet, daß man Polyoxyalkylenmonoether verwendet, bei denen mindestens 50 Mol-% der Oxyalkylen-Einheiten Oxypropylen- und/oder Oxybutylen-Einheiten sind und der durchschnittliche Wert von m bei den übrigen Oxyalkylen-Einheiten 5 bis 14 beträgt.

3. Verfahren nach Anspruch 2, dadurch gekennzeichnet, daß man Polyoxyalkylenmonoether verwendet, bei denen mindestens 90 Mol-% der Oxyalkylen-Einheiten Oxypropylen- und/oder Oxybutylen-Einheiten sind und der durchschnittliche Wert von m bei den übrigen Oxyalkylen-Einheiten 5 bis 14 beträgt.

4. Verfahren nach Anspruch 3, dadurch gekennzeichnet, daß man Polyoxyalkylenmonoether verwendet, bei denen die Oxyalkylen-Einheiten ausschließlich aus Oxypropylen- und/oder Oxybutylen-Einheiten bestehen.

5. Verwendung von (Meth-)acrylsäureestern nach einem der Ansprüche 1 bis 4 als härtbare Monomere im Dentalbereich.

6. Verwendung von (Meth-)acrylsäureestern nach Anspruch 5 als Unterfütterungsmaterial für Zahnprothesen.

## Claims

**Claims for the following Contracting States : AT, BE, CH, DE, FR, GB, GR, IT, LI, LU, NL, SE**

1. Macromonomeric (meth)acrylic acid esters containing fluoroalkyl groups, of the general formula

$$R^1-(C_nF_{2n}-)(CH_2-)_aO-(C_mH_{2m}O-)_b(\underset{\underset{O}{\|}}{C}-NH-(CH_2-)_cO-)\underset{\underset{O}{\|}}{C}-\underset{\underset{R^2}{|}}{C}=CH_2$$

wherein

$R^1$ is identical or different and is a hydrogen or fluorine radical,
$R^2$ is identical or different and is a hydrogen or methyl radical,
a has a value of 0, 1, 2, 3 or 4,
c has a value of 2, 3 or 4,
b has an average value of 2 to 30,
n has an average value of 4 to 12 and
m has an average value of 3 to 14.

2. (Meth)acrylic acid esters according to Claim 1, characterised in that in an average molecule, at least 50 mol% of the oxyalkylene units are oxypropylene and/or oxybutylene units and the average value of m in the other oxyalkylene units is 5 to 14.

3. (Meth)acrylic acid esters according to Claim 2, characterised in that in an average molecule, at least 90 mol% of the oxyalkylene units are oxypropylene and/or oxybutylene units and the average value of m in the other oxyalkylene units is 5 to 14.

4. (Meth)acrylic acid esters according to Claim 3, characterised in that the oxyalkylene units consist exclusively of oxypropylene and/or oxybutylene units.

5. Process for the preparation of (meth) acrylic acid esters according to Claim 1, characterised in that a polyoxyalkylene monoether of the general formula

$$R^1-(C_nF_{2n}-)(CH_2-)_aO-(C_mH_{2m}O-)_bH$$

is reacted with an isocyanate of the general formula

$$OCN-(CH_2)_cO-\underset{\underset{O}{\|}}{C}-\underset{\underset{R^2}{|}}{C}=CH_2$$

wherein the indices have the meaning already given, in a manner which is known per se at temperatures in a range from 20 to 100°C, optionally in the presence of a solvent which is inert towards isocyanate

groups and optionally in the presence of a catalyst which is known per se for reaction of the isocyanate group with the hydroxyl group.

6. Use of (meth)acrylic acid esters according to one of Claims 1 to 4 as hardenable monomers in the dental sector.

7. Use of (meth)acrylic acid esters according to Claim 6 as a lining material for dental prostheses.

**Claims for the following Contracting State : ES**

1. Process for the preparation of macromonomeric (meth)acrylic acid esters containing fluoroalkyl groups, of the general formula

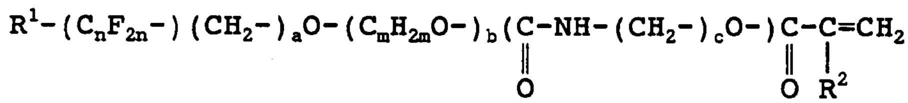

wherein
$R^1$ is identical or different and is a hydrogen or fluorine radical,
$R^2$ is identical or different and is a hydrogen or methyl radical,
a has a value of 0, 1, 2, 3 or 4,
c has a value of 2, 3 or 4,
b has an average value of 2 to 30,
n has an average value of 4 to 12 and
m has an average value of 3 to 14,
characterised in that a polyoxyalkylene monoether of the general formula

$$R^1\text{-}(C_nF_{2n}\text{-})(CH_2\text{-})_aO\text{-}(C_mH_{2m}O\text{-})_bH$$

is reacted with an isocyanate of the general formula

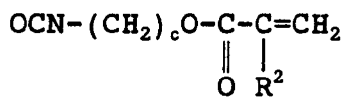

wherein the indices have the meaning already given, in a manner which is known per se at temperatures in a range from 20 to 100°C, optionally in the presence of a solvent which is inert towards isocyanate groups and optionally in the presence of a catalyst which is known per se for reaction of the isocyanate group with the hydroxyl group.

2. Process according to Claim 1, characterised in that in polyoxyalkylene monoethers in which at least 50 mol% of the oxyalkylene units are oxypropylene and/or oxybutylene units and the average value of m in the other oxyalkylene units is 5 to 14 are used.

3. Process according to Claim 2, characterised in that polyoxyalkylene monoethers in which at least 90 mol% of the oxyalkylene units are oxypropylene and/or oxybutylene units and the average value of m in the other oxyalkylene units is 5 to 14 are used.

4. Process according to Claim 3, characterised in that polyoxyalkylene monoethers in which the oxyalkylene units consist exclusively of oxypropylene and/or oxybutylene units are used.

5. Use of (meth)acrylic acid esters according to one of Claims 1 to 4 as hardenable monomers in the dental sector.

6. Use of (meth)acrylic acid esters according to Claim 5 as lining material for dental prostheses.

## Revendications

### Revendications pour les Etats contractants suivants : AT, BE, CH, DE, FR, GB, GR, IT, LI, LU, NL, SE

1. Esters macromonomères de l'acide (méth)acrylique, contenant des groupes fluoroalcoyle et répondant à la formule générale

$$R^1-(C_nF_{2n}-)(CH_2-)_aO-(C_mH_{2m}O-)_b(\underset{\underset{O}{\|}}{C}-NH-(CH_2-)_cO-)\underset{\underset{O}{\|}}{C}-\underset{\underset{R^2}{|}}{C}=CH_2$$

où

$R^1$ est identique ou différent et représente un reste d'hydrogène ou de fluor,
$R^2$ est identique ou différent et représente un reste d'hydrogène ou un reste méthyle,
a vaut 0, 1, 2, 3 ou 4,
c vaut 2, 3 ou 4,
b a une valeur moyenne de 2 à 30,
n a une valeur moyenne de 4 à 12, et
m a une valeur moyenne de 3 à 14.

2. Esters de l'acide (méth)acrylique selon la revendication 1, caractérisés en ce que, dans la molécule moyenne, au moins 50 % en moles des unités oxyalcoylène sont des unités oxypropylène et/ou des unités oxybutylène et la valeur moyenne de m est comprise entre 5 et 14 pour les unités oxyalcoylène restantes.

3. Esters de l'acide (méth)acrylique selon la revendication 2, caractérisés en ce que, dans la molécule moyenne, au moins 90 % en moles des unités oxyalcoylène sont des unités oxypropylène et/ou des unités oxybutylène et la valeur moyenne de m est comprise entre 5 et 14 pour les unités oxyalcoylène restantes.

4. Esters de l'acide (méth)acrylique selon la revendication 3, caractérisés en ce que les unités oxyalcoylène sont formées exclusivement d'unités oxypropylène et/ou d'unités oxybutylène.

5. Procédé de préparation des esters de l'acide (méth)acrylique selon la revendication 1, caractérisé en ce qu'on fait réagir un monoéther de polyoxyalcoylène répondant à la formule générale

$$R^1-(C_nF_{2n}-)(CH_2-)_aO-(C_mH_{2m}O-)_bH$$

avec un isocyanate répondant à la formule générale

$$OCN-(CH_2-)_cO-\underset{\underset{O}{\|}}{C}-\underset{\underset{R^2}{|}}{C}=CH_2$$

où les indices ont chacun la signification indiquée ci-dessus , d'une manière connue en soi, à une température comprise dans la plage de 20 à 100°C, éventuellement en présence d'un solvant inerte vis-à-vis des groupes isocyanates et éventuellement en présence d'un catalyseur connu en soi pour la réaction du groupe isocyanate avec le groupe hydroxyle.

6. Utilisation des esters de l'acide (méth)acrylique selon une des revendications 1 à 4, comme monomères durcissables, dans le domaine dentaire.

7. Utilisation des esters de l'acide (méth)acrylique selon la revendication 6, comme matériau de revêtement interne pour prothèses dentaires.

### Revendications pour l'Etat contractant suivant : ES

1. Procédé de préparation d'esters macromonomères de l'acide (méth)acrylique, contenant des groupes fluoroalcoyle et répondant à la formule générale

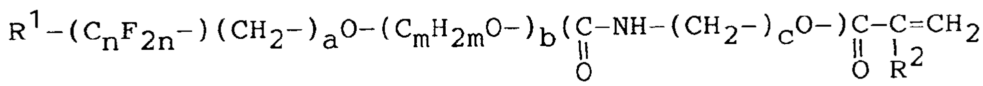

où

$R^1$ est identique ou différent et représente un reste d'hydrogène ou de fluor,
$R^2$ est identique ou différent et représente un reste d'hydrogène ou un reste méthyle,
a vaut 0, 1, 2, 3 ou 4,
c vaut 2, 3 ou 4,
b a une valeur moyenne de 2 à 30,
n a une valeur moyenne de 4 à 12, et
m a une valeur moyenne de 3 à 14,

caractérisé en ce qu'on fait réagir un monoéther de polyoxyalcoylène répondant à la formule générale

$$R^1\text{-}(C_nF2_n\text{-})(CH_2\text{-})_aO\text{-}(C_mH_{2m}O\text{-})_bH$$

avec un isocyanate répondant à la formule générale

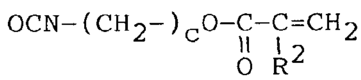

où les indices ont chacun la signification indiquée ci-dessus , d'une manière connue en soi, à une température comprise dans la plage de 20 à 100°C, éventuellement en présence d'un solvant inerte vis-à-vis des groupes isocyanates et éventuellement en présence d'un catalyseur connu en soi pour la réaction du groupe isocyanate avec le groupe hydroxyle.

2. Procédé selon la revendication 1, caractérisé en ce qu'on utilise des monoéthers de polyoxyalcoylène dans lesquels au moins 50 % en moles des unités oxyalcoylène sont des unités oxypropylène et/ou des unités oxybutylène et la valeur moyenne de m est comprise entre 5 et 14 pour les unités oxyalcoylène restantes.

3. Procédé selon la revendication 2, caractérisé en ce qu'on utilise des monoéthers de polyoxyalcoylène dans lesquels au moins 90 % en moles des unités oxyalcoylène sont des unités oxypropylène et/ou des unités oxybutylène et la valeur moyenne de m est comprise entre 5 et 14 pour les unités oxyalcoylène restantes.

4. Procédé selon la revendication 3, caractérisé en ce qu'on utilise des monoéthers de polyoxyalcoylène dans lesquels les unités oxyalcoylène sont formées exclusivement d'unités oxypropylène et/ou d'unités oxybutylène.

5. Utilisation des esters de l'acide (méth)acrylique selon une des revendications 1 à 4, comme monomères durcissables, dans le domaine dentaire.

6. Utilisation des esters de l'acide (méth)acrylique selon la revendication 5, comme matériau de revêtement interne pour prothèses dentaires.